(19) Europäisches Patentamt
European Patent Office
Office européen des brevets

(11) Veröffentlichungsnummer: **0 218 976**
**A2**

# (12) EUROPÄISCHE PATENTANMELDUNG

(21) Anmeldenummer: 86113358.5

(22) Anmeldetag: 29.09.86

(51) Int.Cl.[4]: C 07 D 227/087
C 07 D 223/10, C 07 D 207/2-67

(30) Priorität. 09.10.85 DE 3536028

(43) Veröffentlichungstag der Anmeldung:
22.04.87 Patentblatt 87/17

(84) Benannte Vertragsstaaten:
CH DE FR GB LI

(71) Anmelder: HOECHST AKTIENGESELLSCHAFT
Postfach 80 03 20
D-6230 Frankfurt am Main 80(DE)

(72) Erfinder: Schorr, Manfred, Dr.
Oestricher Weg 10
D-6000 Frankfurt am Main 71(DE)

(72) Erfinder: Schmitt, Wilfried
Im Stückes 35
D-6233 Kelkheim (Taunus)(DE)

## (54) Verfahren zur Herstellung von 1-substituierten Azacycloalkan-2-onen.

(57) Verfahren zur Hestellung von 1-substituierten Azacycloalkan-2-onen der allgemeinen Formel I,

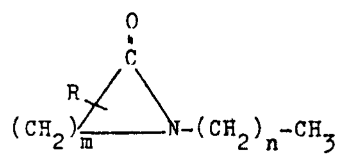
I

in der R Wasserstoff oder niedriges Alkyl mit 1-4 Kohlenstoffatomen, m 3-7 und n 0-17 bedeuten, dadurch gekennzeichnet, daß man ein 1-Trimethylsilyl-azacycloalkan-2-on der allgemeinen Form ...

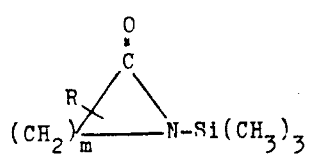
III

in der R und m die angegebene Bedeutung besitzen, mit einem Alkalimetallalkoholat oder einem Alkalimentalloxid umsetzt und zur Einführung des Alkylrestes $-(CH_2)_n-CH_3$ auf das gebildete Alkalimetallsalz des Azacycloalkan-2-ons in an sich bekannter Weise ein Alkylierungsmittel einwirken läßt.

Verfahren nach Anspruch 1, dadurch gekennzeichnet, daß man als Alkalimetallalkoholat Natriummethylat oder Natriumäthylat verwendet.

Verfahren nach Anspruch 1, dadurch gekennzeichnet, daß man als Alkalimetalloxid Natriumoxid verwendet.

Verfahren zur Herstellung von 1-Substituierten Azacycloalkan-2-onen der Formel I

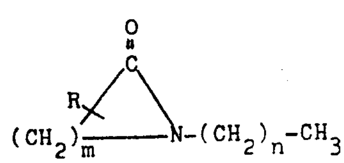
I

worin R, m und n die angegebenen Bedeutungen haben durch Umsetzung von entsprechenden 1-Trimethylsilylazacycloalkan-2-onen mit Alkalimetallalkoholat oder Alkalimetalloxid und anschließende Alkylierung der gebildeten Alkalimetallsalze.

EP 0 218 976 A2

# Verfahren zur Herstellung von 1-substituierten Azacycloalkan-2-onen

Es ist bekannt, daß 1-substituierte Azacycloalkan-2-one der allgemeinen Formel I,

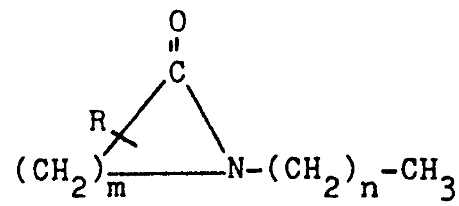

I

in der R Wasserstoff oder niedriges Alkyl mit 1-4 Kohlenstoffatomen, m 3-7 und n 0-17 bedeuten, die Penetrationsfähigkeit pharmazeutischer Wirkstoffe durch die Haut erhöhen. So werden in den US-Patenten 3 989 816, 4 316 893 und 4 405 616 pharmazeutische Zubereitungen beschrieben, die infolge ihres Gehalts an Verbindungen der Formel I für die transdermale Applikation von Arzneimitteln geeignet sind. Darüber hinaus wird im US-Patent 4 311 481 gezeigt, daß 1-substituierte Azacycloalkan-2-one der angegebenen Struktur das Eindringen von Farbstoffen in Textilfasern begünstigen und damit den Färbevorgang verbessern.

Die Herstellung der Verbindungen der allgemeinen Formel I erfolgt im allgemeinen durch Alkylierung von 1-unsubstituierten Azacycloalkan-2-onen der allgemeinen Formel II,

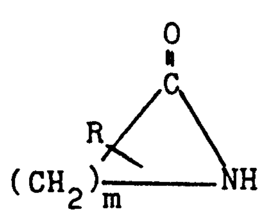

II

in der R und m die obengenannte Bedeutung haben, durch Einwirkung eines Alkylierungsmittels, wie z.B. eines Alkylhalogenids oder eines Alkylsulfonats. Dabei ist es erforderlich, das Azacycloalkan-2-on vorher in sein Anion zu überführen. Das wird durch Umsetzung mit Natriummetall [Helv.Chim.Acta 4 (1921), 480] oder Natriumhydrid bzw. Natriumamid (US-Patent 4 316 893) erreicht. Der Umgang mit diesen Reagenzien erfordert wegen ihrer großen Reaktionsfähigkeit, vor allem gegenüber Feuchtigkeit, besondere Vorsichts- und Schutzmaßnahmen, weshalb sich das Arbeiten, vorzugsweise im technischen Maßstab, schwierig und aufwendig gestaltet.

In Chem.Ber. 102, (1969), 3094, wird beschrieben, daß sich Natriumsalze von Carbonsäureamiden dadurch gewinnen lassen, daß man auf die entsprechenden N-Trimethylsilylderivate Natriumsilanolat einwirken läßt. Die besonders hohe Bindungsenergie der Silizium-Sauerstoff-Bindung in Disiloxanen führt zur Bildung von Hexamethyldisiloxan, wobei gleichzeitig das Amid-Natriumsalz entsteht. Dieses einfache Verfahren ist jedoch auf die Anwendung im Labormaßstab beschränkt. Die schwere Zugänglichkeit und der damit verbundene hohe Preis des Natriumsilanolats verbietet seinen Einsatz in großtechnischen Prozessen.

Es wurde nun überraschenderweise gefunden, daß man 1-substituierte Azacycloalkan-2-one der allgemeinen Formel I in einfacher Weise herstellen kann, indem man die entsprechenden 1-Trimethylsilylverbindungen mit Alkalimetallalkoholat oder Alkalimetalloxid umsetzt und anschließend alkyliert.

Die Erfindung betrifft daher ein Verfahren zur Herstellung der Verbindungen der Formel I

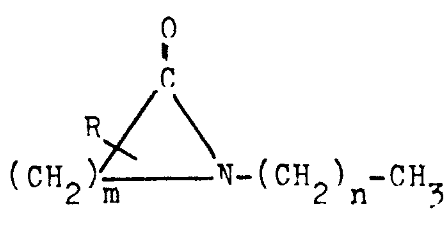

I

worin R, m und n die angegebenen Bedeutungen haben, welches dadurch gekennzeichnet ist, daß man 1-Trimethylsilylazacycloalkan-2-one der allgemeinen Formel III,

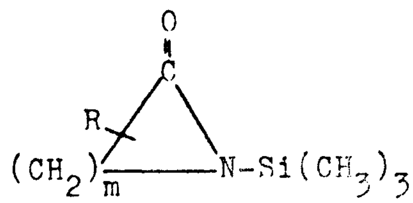

III

in der R und m die oben angegebenen Bedeutungen besitzen, mit einem Alkalimetallalkoholat oder einem Alkalimetalloxid umsetzt und zur Einführung des Alkylrestes-$(CH_2)_nCH_3$ auf das gebildete Alkalimetallsalz des Azacycloalkan-2-ons in an sich bekannter Weise ein Alkylierungsmittel einwirken läßt. Dabei ist es nicht notwendig, das Alklimetallsalz zu isolieren, so daß die Reaktion fortlaufend in einem Gefäß durchgeführt werden kann.

Die als Ausgangsmaterial eingesetzten N-Trimethylsilylazacycloalkan-2-one können in einfacher und billiger Weise aus den unsubstituierten Azacycloalkan-2-onen der allgemeinen Formel II durch Reaktion mit Trimethylchlorsilan in Gegenwart von Triäthylamin [Chem.Ber. 99, 3820 (1966)] oder mit Hexamethyldisilazan (EP-A-00 43 630) hergestellt werden.

Als Alkalimetallalkoholate oder Alkalimetalloxide, die im ersten Schritt eingesetzt werden, kommen vorzugsweise die Alkoholate oder Oxide von Lithium, Natrium oder Kalium in Betracht. Besonders vorteilhaft verwendet man Natriummethylat, Natriumäthylat oder Natriumoxid. Diese leicht zugänglichen und wohlfeilen Reagenzien lassen sich auch im großtechnischen Maßstab gefahrlos und ohne zusätzlichen Aufwand und besondere Vorsichtsmaßnahmen handhaben.

Die Umsetzungen werden vorteilhaft in Gegenwart eines inerten Lösungsmittels durchgeführt. Besonders geeignet

sind aromatische Kohlenwasserstoffe, wie z.B. Benzol oder Toluol, oder aliphatische oder aromatische Äther, wie z.B. 1,2-Dimethoxyäthan oder Anisol.

Die Durchführung der Reaktion erfolgt zweckmäßig in der Weise, daß man zunächst das Alkalimetallalkoholat oder -oxid in dem inerten Lösungsmittel suspendiert und dann das 1-Trimethylsilyl-azacycloalkan-2-on, gegebenenfalls gelöst in dem gleichen Lösungsmittel, zufügt. Die Bildung des Alkalimetallsalzes des Azacycloalkan-2-ons findet bereits bei Raumtemperatur statt. Zur Beschleunigung und Vervollständigung der Umsetzung ist es jedoch oft vorteilhaft, auf höhere Temperaturen, z.B. bis zur Siedetemperatur des verwendeten Lösungsmittels, zu erhitzen.

Das Reaktionsgemisch wird anschließend, gegebenenfalls nach vorhergehendem Abkühlen, mit dem Alkylierungsmittel versezt. Als solche kommen Alkylhalogenide oder Ester von Akoholen mit Schwefelsäure oder aliphatischen oder aromatischen Sulfonsäuren in Betracht. Die Umsetzung mit dem Natriumsalz kann schon bei Raumtemperatur stattfinden. Im allgemeinen ist es jedoch vorteilhaft, bei erhöhter Temperatur, vorzugsweise zwischen 50 und 150°C, zu arbeiten.

Nach Beendigung der Reaktion filtriert man von der gebildeten Alkalimetallverbindung, wie Alkalimetallhalogenid bzw. -sulfonat, ab und entfernt das Lösungsmittel durch Abdestillieren. Die 1-substituierten Azacycloalkan-2-one bleiben im allgemeinen als Öle zurück und können durch Destillation im Vakuum in reiner Form erhalten werden.

Die Aufarbeitung des Reaktionsgemischs kann aber auch in der Weise erfolgen, daß man zunächst das Lösungsmittel i.V. abdestilliert, den Rückstand mit Wasser und einem mit Wasser nicht mischbaren Lösungsmittel behandelt, letzteres abtrennt, trocknet und abdampft. Das zurückbleibende Öl wird dann i.V. destilliert.

Beispiel 1: N-n-Dodecylcaprolactam

Zu einer Suspension von 10,8 g (0,2 M) Natriummethylat in 300 ml Toluol werden bei Raumtemperatur 37 g (0,2 M) N-Trimethylsilylcaprolactam getropft und weitere 30 Minuten gerührt. Dabei bildet sich eine dicke Suspension des Caprolactam-natriumsalzes. Nach Zugabe von 49,8 g (0,2 M) n-Dodecylbromid wird das Reaktionsgemisch 20 Stunden unter Rückfluß erhitzt und gerührt. Anschließend wird das Toluol i.V. abdestilliert, der Rückstand mit Diäthyläther und Wasser aufgenommen, die Ätherphase abgetrennt, viermal mit Wasser gewaschen und über Natriumsulfat getrocknet. Nach dem Abdestillieren des Äthers bleibt ein Öl zurück, das im Vakuum destilliert wird.
$Kp_{0,17\ mm}$ 182-184°C; Ausbeute 39,7 g

Beispiel 2: N-n-Dodecylcaprolactam

Umsetzung analog Beispiel 1; anstelle von 300 ml Toluol werden 300 ml 1,2-Dimethoxyäthan als Lösungsmittel eingsetzt.
$Kp_{0,15\ mm}$ 175-176°C; Ausbeute 39,5 g

Beispiel 3: N-n-Dodecylcaprolactam

3,1 g (0,05 M) Natriumoxid ($Na_2O$) werden in 150 ml 1,2-Dimethoxyäthan suspendiert und dann 18,5 g (0,1 m) N-Trimethylsilylcaprolactam zugesetzt. Das Reaktionsgemisch wird 2 Stunden bei 50°C gerührt, wobei sich der voluminöse Niederschlag des Caprolactam-natriumsalzes bildet. Anschließend werden 24,9 g (0,1 M) n-Dodecylbromid zugefügt und 20 Stunden unter Rückfluß erhitzt und gerührt. Die Aufarbeitung erfolgt wie in Beispiel 1 angegeben.
$Kp_{0,15\ mm}$ 175-176°C; Ausbeute 20,8 g

Beispiel 4: N-Methylcaprolactam

In 150 ml 1,2-Dimethoxyäthan werden nacheinander 5,4 g (0,1 M) Natriummethylat und 18,5 g (0,1 M) N-Trimethylsilylcaprolactam eingetragen und 30 Minuten bei Raumtemperatur gerührt. Anschließend werden 11 g (0,1 M) Methansulfonsäuremethylester zugetropft. Nachdem man noch 1 Stunde bei 50°C gerührt hat, wird das Reaktionsgemisch abgekühlt, von Natriummesylat abgesaugt und das Filtrat eingedampft. Der ölige Rückstand wird im Vakuum destilliert.
$Kp_{6\ mm}$ 100-102°C; Ausbeute 8,9 g

Beispiel 5: 1-n-Hexylpyrrolidin-2-on

Zu einer Suspension von 10,8 g (0,2 M) Natriummethylat in 200 ml 1,2-Dimethoxyäthan werden rasch 31,4 g (0,2 M) 1-Trimethylsilylpyrrolidin-2-on gegeben. Zur Vervollständigung der Umsetzung erhitzt man noch 2 Stunden unter Rückfluß, kühlt das Reaktionsgemisch dann ab und tropft 31 g (0,2 M) n-Hexylbromid zu. Anschließend wird 8 Stunden unter Rückfuß erhitzt, abgekühlt und von entstehendem Natriumbromid abgesaugt. Nach dem Eindampfen des Filtrats bleibt ein Öl zurück, das i.V. destilliert wird.
$Kp_{0,35\ mm}$ 91-93°C; Ausbeute 26 g

Patentansprüche:

1) Verfahren zur Herstellung von 1-substituierten Azacycloalkan-2-onen der allgemeinen Formel I,

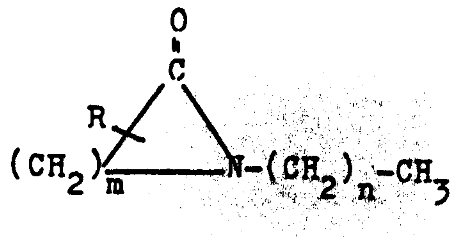

I

in der R Wasserstoff oder niedriges Alkyl mit 1-4 Kohlenstoffatomen, m 3-7 und n 0-17 bedeuten, dadurch gekennzeichnet, daß man ein 1-Trimethylsilyl-azacycloalkan-2-on der allgemeinen Formel III,

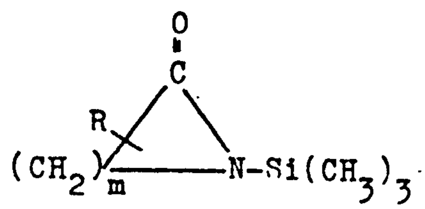

III

in der R und m die angegebene Bedeutung besitzen, mit einem Alkalimetallalkoholat oder einem Alkalimetalloxid umsetzt und zur Einführung des Alkylrestes $-(CH_2)_n-CH_3$ auf das gebildete Alkalimetallsalz des Azacycloalkan-2-ons in an sich bekannter Weise ein Alkylierungsmittel einwirken läßt.

2) Verfahren nach Anspruch 1, dadurch gekennzeichnet, daß man als Alkalimetallalkoholat Natriummethylat oder Natriumäthylat verwendet.

3) Verfahren nach Anspruch 1, dadurch gekennzeichnet, daß man als Alkalimetalloxid Natriumoxid verwendet.